# DEMANDE DE BREVET EUROPEEN

(11) **EP 0 536 055 A1**
(43) Date de publication de la demande: **07.04.1993**
(21) Numéro de dépôt: 92402698.2
(22) Date de dépôt: 02.10.1992
(51) Int. Cl.: A43B 7/28, A43D 1/02

(54) **Procédé et dispositif pour l'obtention d'une empreinte de la surface d'un corp**

(30) Priorité: 03.10.1991 FR 9112196
(71) Demandeur: ETABLISSEMENTS SALEMBIER FRERES (SA), F-59000 Lille (FR)
(72) Inventeur: Leonard, Michel, F-21000 Dijon (FR)
(74) Mandataire: Bruder, Michel

(57) **Abrégé**

La présente invention concerne un procédé, divers dispositifs ainsi qu'un appareillage pour l'obtention d'une empreinte de la surface d'un objet (4) ou d'une partie du corps.

Le procédé, par lequel une matière modelable (3) est disposée entre ladite surface et un côté d'une membrane déformable (2), susceptible d'épouser la forme de ladite surface sous l'effet de la pression qu'y exerce ledit objet (4) ou ladite partie du corps, est caractérisé en ce que des moyens pneumatiques commandés sont agencés de l'autre côté de ladite membrane (2) pour délivrer une contrepression continûment variable permettant de contrôler en permanence l'amplitude des déformations de ladite matière modelable (3).

L'invention est notamment destinée à la réalisation de semelles anatomiques ou correctrices.

## Description

La présente invention concerne un procédé, divers dispositifs ainsi qu'un appareillage pour l'obtention d'une empreinte de la surface d'un objet ou d'une partie du corps. L'invention est notamment destinée à la réalisation de semelles anatomiques ou correctrices.

On connaît, par les brevets US-4 962 593 et US-4 503 576, diverses techniques de réalisation de semelles anatomiques reproduisant l'empreinte d'un pied, selon lesquelles on crée un vide dans un sac entourant ledit pied préalablement positionné sur un insert déformable. De même, dans le brevet FR-2 621 237, un jeu d'enceintes superposées qui sont remplies d'un matériau granuleux (technique dite du "lit fluidisé") se rigidifient lorsque l'on y crée le vide, ce qui permet d'obtenir l'empreinte du pied. A l'inverse, dans les brevets FR-2 335 171, on a proposé un dispositif par lequel, pour fabriquer une semelle, une feuille en matière thermoplastique ramollie est comprimée entre le pied d'une personne et une surface élastique, pouvant être une enveloppe contenant un liquide. De même, dans le brevet FR-2 435 217, une enveloppe souple est refermée autour de la feuille en matière thermoplastique ramollie et du pied, avant d'être gonflée. Enfin, dans le brevet FR-1 465 979, une plaque en matière thermoplastique ramollie est placée sur un coussin contenant une masse de caoutchouc mousse et d'un liquide dont la quantité peut varier.

Aucun de ces dispositifs ne procure le moyen de contrôler et de connaître en permanence la pression s'exerçant sur la feuille thermoplastique ou thermodurcissable employée pour réaliser la semelle ; en conséquence, tous présentent des inconvénients notables quant à la fidélité de la reproduction que l'on peut obtenir du dessous du pied. En particulier, la technique de prise d'empreinte par lit fluidisé est une méthode statique proposant deux positions alternatives extrêmes et ne permettant donc pas de reprise sur la semelle fabriquée en cours d'opération ; en outre, cette technique provoque une mise en position vers le haut des orteils, ce qui interdit souvent la réalisation d'une semelle entière jusque sous ces mêmes orteils. D'un autre côté, les sacs dans lesquels on crée le vide ne donnent qu'un aperçu médiocre du pied en action, et il est en particulier connu qu'une semelle réalisée dans ces conditions ne tient pas compte de la posture du pied en position dynamique de marche. De même, les poches remplies d'un fluide incompressible, même à volume variable, ne permettent pas de tenir compte, d'une manière connue et parfaitement contrôlée, des pressions exactes exercées par le pied de la personne, ni de les corriger par l'apport d'éléments correctifs distribués sous la semelle ; le résultat obtenu est donc aléatoire et ne permet pas aux podologues de traiter correctement certaines déformations.

D'une manière plus générale, la prise d'empreinte d'objets de toutes sortes pose le problème de la fidélité de reproduction ainsi que du contrôle permanent, en cours de moulage, du résultat obtenu ou recherché.

La présente invention vise à remédier à ces inconvénients en proposant un procédé d'obtention d'une empreinte de la surface d'un objet ou d'une partie du corps au moyen d'une matière modelable qui est disposée entre ladite surface et un côté d'une membrane déformable, susceptible d'épouser la forme de ladite surface sous l'effet de la pression qu'y exerce ledit objet ou ladite partie du corps, procédé caractérisé en ce que des moyens pneumatiques commandés sont agencés de l'autre côté de ladite membrane pour délivrer une contrepression continûment variable permettant de contrôler en permanence l'amplitude des déformations de ladite matière modelable.

Un premier objectif de la présente invention est ainsi de procurer un moyen pour obtenir le relief exact d'un objet, ou d'une partie du corps telle que le pied, d'une manière parfaitement contrôlable.

Un autre objectif de l'invention consiste en ce que l'on puisse obtenir les reliefs voulus modifiant l'empreinte exacte d'un objet, ou d'une partie du corps telle que le pied, par exemple dans un but thérapeutique ; dans ce cas, il est essentiel de pouvoir tenir compte du poids de la personne et de son tonus musculaire, ce que permet l'invention par le jeu du contrôle de la contrepression pouvant être exercée sous le pied.

En particulier, suivant une autre caractéristique de l'invention, la distribution spatiale des moyens pneumatiques commandés, relativement à la surface de la membrane déformable, peut être choisie en fonction de la distribution locale des pressions exercées par l'objet ou la partie du corps à reproduire. A cet égard, des moyens pneumatiques commandés susceptibles de délivrer une contrepression de forte amplitude seront préférentiellement agencés sous les zones de l'objet ou de la partie du corps à reproduire qui exercent les plus faibles pressions sur la membrane déformable, tandis qu'inversement, des moyens pneumatiques commandés susceptibles de délivrer une contrepression de plus faible amplitude seront agencés sous les zones dudit objet ou de ladite partie du corps à reproduire qui exercent les plus fortes pressions sur ladite membrane déformable.

L'invention concerne également un dispositif pour la réalisation du procédé précédent, caractérisé en ce que les moyens pneumatiques commandés sont constitués par une poche flexible fermée à volume variable dans laquelle peut être introduit un fluide compressible, par exemple un gaz ou un mélange gazeux.

En particulier, dans le cas de l'obtention de l'empreinte de la voûte plantaire du pied d'une personne, une matière modelable est disposée entre ladite voûte et un côté de la membrane déformable susceptible d'épouser la forme de ladite voûte sous l'effet de la pression qu'y exerce ledit pied, et au moins une chambre à air à volume variable est agencée de l'autre côté de ladite membrane ; selon l'invention, chaque chambre est reliée à un moyen de gonflage muni d'un manomètre de sorte à faire varier, tout en la contrôlant, la pression régnant dans ladite chambre.

Suivant une autre caractéristique, chaque chambre à air est susceptible d'être gonflée entre une pression minimale et une pression maximale dont le rapport est supérieur ou égal à 20 ; on comprend donc qu'il soit possible de varier considérablement l'enfoncement du pied, avec l'avantage de disposer en permanence d'une valeur de pression représentative du poids et du tonus musculaire de la personne. A cet égard, on pourra établir des abaques poids/pression destinées à faciliter l'emploi d'un dispositif de prise d'empreinte conforme à l'invention ; ainsi, l'obtention de reliefs forts ou faibles sur une semelle anatomique ou correctrice est indépendante du poids du sujet.

De même si on recherchait à obtenir l'empreinte d'un objet quelconque indépendamment de son poids.

Suivant une première variante de l'invention, plus particulièrement destinée à la podologie, le dispositif de prise d'empreinte comporte une seule chambre à air constituée par un tore de révolution, ou par un simple secteur torique.

Dans ce cas, la membrane déformable sur laquelle le pied comprime la matière modelable de prise d'empreinte sera avantageusement disposée pour que le pied puisse être tangent au milieu de l'arc médian des arcs intérieur et extérieur de ladite chambre, la partie externe du pied étant située du côté de l'arc intérieur ; à cet égard, on définit l'arc intérieur de la chambre comme l'arc du tore situé du côté distal du pied, et inversement, l'arc extérieur de la chambre comme l'arc du tore situé du côté proximal du pied. De cette façon, étant donné que la pression partielle régnant dans la chambre du côté de son arc extérieur est plus forte que la pression partielle du côté de son arc intérieur (effet connu en soi), il est possible d'obtenir une contrepression de faible amplitude sous la partie externe du pied qui exerce les plus fortes pressions sur la membrane déformable et la matière modelable de prise d'empreinte, et, inversement, d'obtenir une contrepression de plus forte amplitude sous la partie interne du pied où la voûte plantaire est normalement plus éloignée de ladite membrane.

On a pu établir, dans ce cas, que la géométrie et les dimensions nominales du tore ou du secteur torique devaient être les suivantes :
- le tore ou le secteur torique est de section droite circulaire, ovale ou oblongue,
- le rayon intérieur est au moins égal à 40 centimètres,
- le rayon extérieur est au moins égal à 65 centimètres,
- et, dans le cas, du secteur torique, la longueur moyenne, prise au niveau de l'arc médian des arcs intérieur et extérieur, est au moins égale à 40 centimètres.

De telle chambres à air sont couramment disponibles dans le commerce à l'usage des roues des véhicules poids lourds.

Dans une seconde variante, le dispositif de prise d'empreinte comporte deux chambres à air, interne et externe, maintenues côte à côte, chaque chambre pouvant être gonflée de manière autonome pour solliciter la membrane déformable symétriquement par rapport à son axe médian ; cette disposition des deux chambres procure un autre moyen également très simple pour contrebalancer séparément la pression exercée par la partie interne et la partie externe du pied sur ladite membrane. En particulier, il devient possible d'inverser le sens des contrepressions hautes et basses agissant sous le pied, ce qui procure un moyen de tenir compte des déformations latérales du pied dites en pronation, et, à l'inverse, des déformations du pied dites en supination.

Par ailleurs, bien que, dans le cas d'utilisation de deux chambres juxtaposées, on puisse tout à fait se contenter de chambres cylindriques, il est encore possible que les deux chambres à air, interne et externe, soient constituées par des tores ou des secteurs toriques de révolution, de sections droites circulaires, ovales ou oblongues, normalement prises identiques, le rayon intérieur de la chambre torique placée du côté distal du pied étant au moins égal à 40 centimètres, le rayon extérieur de la chambre torique plcée du côté proximal du pied étant au moins égal à 65 centimètres, et la longueur de l'arc séparant les deux chambres étant au moins égale à 40 centimètres.

D'autres caractéristiques et avantages de la présente invention ressortiront mieux de la description qui va suivre d'un mode d'exécution d'un appareillage pour la fabrication de semelles anatomiques ou correctives adaptées aux pieds d'une personne, ce mode d'exécution étant donné à titre d'exemple non limitatif en référence aux dessins annexés sur lesquels :
- la figure 1 est une vue en perspective montrant schématiquement une chambre à air toroïdale, munie d'une valve, d'un gonfleur et d'un manomètre, sur laquelle on a disposé une membrane souple et un objet dont l'embase est à reproduire,
- la figure 2 est une vue schématique de dessus de l'appareil préconisé,
- la figure 3 est une vue en coupe transversale de l'appareillage prise au niveau d'un caisson,
- la figure 4 est une vue similaire à la figure 3, montrant une variante de réalisation,
- les figures 5 à 7 sont des vues longitudinales d'un pied reposant sur une membrane déformable, agencée au dessus d'une chambre à air à la manière de l'invention, montrant les étapes de prise d'empreinte du dessous du pied et, plus particulièrement, le contrôle de l'enfoncement de ce même pied en fonction du résultat à atteindre.

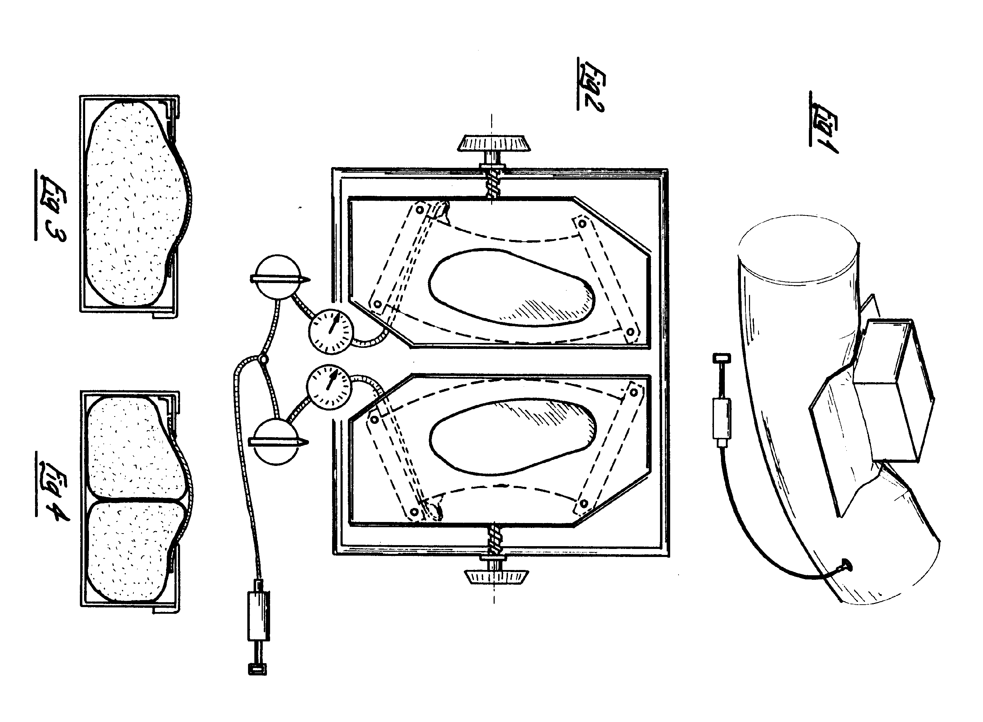

Suivant la figure 1, une chambre à air 1, qui peut être torique, est agencée sous une membrane déformable 2 pour supporter, le cas échant au travers d'une feuille d'une matière modelable 3, un objet 4, par exemple parallélipipèdique, dont on veut réaliser l'empreinte. A cet effet, la chambre à air 1 est munie d'un dispositif de gonflage, ou gonfleur 5, d'un type courant, lequel est relié à une valve 6 au travers d'un manomètre 7 de contrôle de la pression moyenne régnant à l'intérieur de ladite chambre à air 1. Pour obtenir l'empreinte de l'embase de l'objet 4, on gonfle la chambre à air 1 à une pression donnée, contrôlée par le manomètre 7 ; puis, on dispose l'objet4 sur la membrane 2 qui s'enfonce alors dans la chambre 1 en y élevant la pression ; par le jeu du gonfleur 5, il est aisé d'adapter l'amplitude de cet enfoncement, soit en l'augmentant par un échappement contrôlé d'air, soit en le diminuant par un surgonflage de la chambre 1. Dans tous les cas, l'enfoncement ne dépend que de la pression lue sur le manomètre 7, et non du poids de l'objet 4.

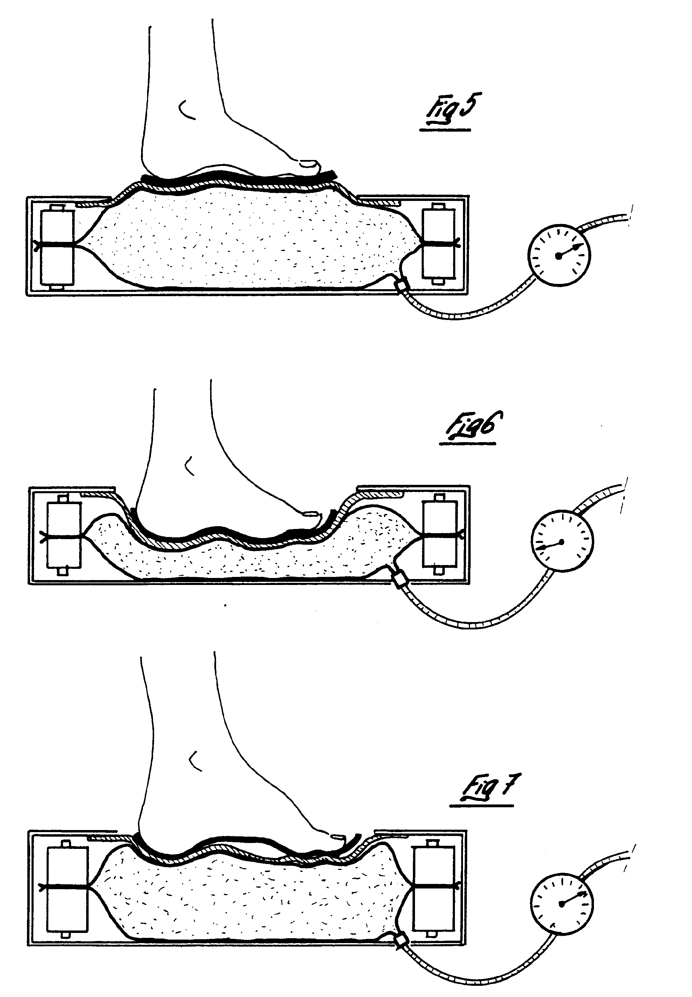

On décrira maintenant, en référence aux figures 2 à 7, l'application de cette technique de prise d'empreinte contrôlée à un appareillage pour la fabrication de semelles anatomiques ou correctives adaptées aux pieds d'une personne.

Suivant tout d'abord la figure 2, l'appareil proposé comporte deux dispositifs symétriques l'un de l'autre par rapport à un axe médian, qui sont respectivement destinés à l'obtention de l'empreinte de la voûte plantaire du pied droit et du pied gauche d'une personne.

Chacun de ces dispositifs comporte :
- au moins une chambre à air 1, en forme de secteur torique, qui est agencée, à l'intérieur d'un caisson 8, sous une membrane déformable 2, par exemple en caoutchouc blond naturel, sur laquelle on a disposé une matière modelable 3, telle que par exemple une semelle thermoformable de base en résine, cette semelle ayant été préalablamenent ramollie par chauffage au four, ou trempage dans de l'eau bouillante ;
- un moyen de gonflage, ou gonfleur 5, muni d'un manomètre 7, de sorte à faire varier, tout en la contrôlant, la pression régnant dans la chambre à air 1.

On observera que, suivant le procédé de prise d'empreinte conforme à la présente invention, chaque chambre à air 1 est susceptible d'être gonflée entre une pression minimale égale à 20 millibars (soit 2000 Pa) et une pression maximale égale à 400 millibars (soit 40000 Pa). On rappelle également qu'il est possible d'agencer plus d'une seule chambre à air 1 sous la membrane déformable 2 (Cf. figure 3), par exemple deux chambres à air 1a, 1b, le cas échant toroïdales, munies chacune d'un gonfleur 5 et d'un manomètre 7 (Cf. figure 4).

Selon la variante représentée sur les dessins, la membrane déformable 2 - sur laquelle est posée la semelle thermoformable de base destinée à reproduire la surface de la voûte plantaire du pied concerné - est tendue devant un ajourage oblong 9 qui est pratiqué dans la face supérieure du caisson 8 contenant la chambre à air 1 ; selon l'invention, l'axe médian dudit ajourage 9 est orienté sur le caisson 8 pour être sensiblement tangent au milieu de l'arc médian des arcs intérieur et extérieur de ladite chambre toroïdale 1. Dans l'exemple représente, cet axe médian est orienté à environ 15 ° par rapport aux bords verticaux du caisson 8 ; de ce fait, les axes médians des ajourages oblongs 9 des deux caissons 8 équipant respectivement les dispositifs pour l'obtention de l'empreinte de la voûte plantaire du pied gauche et du pied droit sont orientés de 30 ⁰ l'un par rapport à l'autre.

En outre, l'ajourage oblong 9 de chaque caisson 8 est plus grand que la semelle correspondant à la pointure de pieds la plus élevée, soit légèrement moins que la longueur nominale de la chambre à air 1 lui correspondant ; de cette manière, la chambre 1 une fois gonflée présente un léger effet de hernie, centrée sur l'ajourage 9, qui contribue à la stabilisation de ladite chambre 1, et, par conséquent, à la fidélité de reproduction de la prise d'empreinte du pied concerné. Nominativement, la longueur des ajourages 9 est de 34,5 cm pour une largeur maximale de 13,5 cm au niveau de l'avant-pied.

Par ailleurs, des éléments de correction 10 peuvent être intercalés entre la semelle thermoformable de base en résine et la membrane déformable 2. Ces éléments de correction 10, tout à fait classiques, peuvent être ou non thermoformés en même temps que la semelle. Il est donc simple, pour le praticien, de choisir les gabarits ou les éléments de correction 10 qu'il considère comme adaptés à la déformation dont souffre son patient pour réaliser, d'une manière parfaitement contrôlée, une semelle corrective ne dépendant en aucune manière du poids dudit patient, mais uniquement des reliefs anatomiques ou correctifs souhaités.

A cet égard, on comprend qu'il soit nécessaire qu'aussi bien le pied du patient, que la semelle de base et les éléments correctifs 10 éventuellement employés, soient positionnés avec exactitude les uns par rapport aux autres ; c'est pourquoi il est prévu, conformément à la présente invention, que la zone de la membrane déformable 2 délimitée par l'ajourage 9 soit pourvue d'un repérage longitudinal millimétrique pour le positionnement du pied, de la semelle de base et/ou des éléments de correction 10.

Entre autres avantages, l'appareillage conforme à la figure 2 permet d'obtenir des reliefs et des corrections différentes pour le pied droit et pour le pied gauche, cet avantage résultant de la totale indépendance des variations de pression pouvant être obtenues dans chacune des chambres à air 1 des deux caissons 8.

En outre, il est prévu que les deux caissons 8 soient montés dans un support commun 11, par exemple du type d'une mallette refermable et transportable, ce support 11 étant muni de moyens tels que des vis hélicoïdales 12 permettant d'écarter les deux caissons 8 l'un de l'autre pour tenir compte de l'écartement des deux pieds du patient (et donc de sa corpulence).

L'appareillage décrit s'emploie de la manière suivante : après gonflage des chambres à air 1 à une pression moyenne tenant compte principalement de la pointure, du type de pied, creux ou faible, ainsi que du poids du patient, ce dernier monte debout sur les caissons 8, ses pieds étant respectivement posés sur les ajourages oblongs 9 pratiqués sur la face supérieure respective desdits caissons 8. A cet effet, l'appareillage comporte deux cannes verticales amovibles, non représentées sur les figures, permettant au patient de se tenir ; en effet, celui-ci se trouve alors en appui instable sur les deux chambres à air 1 gonflées.

Cette position très particulière de recherche d'équilibre, combinée au contrôle des pressions régnant dans les deux chambres 1, constitue un aspect essentiel et innovant de la présente invention, présentant les avantages suivants :
- les reliefs latéraux des deux semelles sont naturellement obtenus, d'une part, grâce à l'enfoncement variable des pieds dans leur membrane déformable 2 respective, et, d'autre part, grâce à l'effet dynamique résultant de l'instabilité relative du pied sur ladite membrane 2 ;
- la position d'un pied sur la membrane 2 est parfaitement contrôlée par le praticien qui peut, par un simple déplacement de ses repères, agir pour créer un effet en talus ou un effet équin ; ce même effet pourra être créé par la simple adaptation de la posture antéropostérieure du patient, ce qui va entraîner un changement de la position du pied ;
- les pieds sont libres de toute contrainte, ce qui permet, contrairement à toutes les techniques connues dans l'art antérieur, de conserver un certain dynamisme antéropostérieur et latéral du pied ;
- les appuis des pieds sur les deux membranes 2, recouvertes de semelles de bases ramollies, sont réalisés naturellement par le poids du corps du patient, compensé de la manière décrite plus haut ;
- la posture du patient, en position debout, est la position naturelle de fonctionnement podale ; en conséquence, les semelles obtenues sont très confortables et maintiennent parfaitement les pieds dans leur déroulement spécifique à la marche.

Selon l'invention, une abaque, ou tableau, accompagne normalement l'appareillage proposé pour donner, à titre indicatif, les pressions conseillées en fonction du poids du patient, de sa pointure et de son type de pieds. Les pressions conseillées sur cet abaque permettent l'obtention de semelles offrant un relief considéré comme normal, le praticien restant libre d'utiliser des pressions différentes pourforcer plus ou moins les reliefs, notamment latéraux, que prendront les semelles. Diverses situations d'enfoncement du pied sont représentées sur les figures 5 à 7, le pied étant peu enfoncé sur la figure 5, et très enfoncé sur la figure 6, la figure 7 montrant la réalisation d'une semelle avec interposition, au moment de la prise d'empreinte, d'un gabarit ou d'un élément de correction 10 entre la semelle de base et la membrane déformable 2.

L'invention n'est pas limitée par la description particulière de l'appareillage précédent mais s'étend à tout dispositif analogue, destiné ou non à la podologie. En particulier, la chambre à air avec dispositif de gonflage et manomètre pourrait être remplacée par tout autre moyen pneumatique commandé, y compris par un jeu de vérins agencés verticalement pour supporter l'objet à reproduire.

## Revendications

1 - Procédé d'obtention d'une empreinte de la surface d'un objet (4) ou d'une partie du corps au moyen d'une matière modelable (3) qui est disposée entre ladite surface et un côté d'une membrane déformable (2), susceptible d'épouser la forme de ladite surface sous l'effet de la pression qu'y exerce ledit objet (4) ou ladite partie du corps, procédé caractérisé en ce que des moyens pneumatiques commandés sont agencés de l'autre côté de ladite membrane (2) pour délivrer une contrepression continûment variable permettant de contrôler en permanence l'amplitude des déformations de ladite matière modelable (3).

2 - Procédé d'obtention d'une empreinte selon la revendication 1, caractérisée en ce que la distribution spatiale des moyens pneumatiques commandés, relativement à la surface de la membrane déformable (2), est choisie en fonction de la distribution locale des pressions exercées par l'objet (4) ou la partie du corps à reproduire.

3 - Procédé d'obtention d'une empreinte selon la revendication 2, caractérisée en ce que des moyens pneumatiques commandés susceptibles de délivrer une contrepression de forte amplitude sont agencés sous les zones de l'objet (4) ou de la partie du corps à reproduire qui exercent les plus faibles pressions sur la membrane déformable (2), et, inversement, en ce que des moyens pneumatiques commandés susceptibles de délivrer une contrepression de plus faible amplitude sont agencés sous les zones dudit objet (4) ou de ladite partie du corps à reproduire qui exercent les plus fortes pressions sur ladite membrane déformable (2).

4 - Dispositif pour l'obtention d'une empreinte de la surface d'un objet (4) ou d'une partie du corps au moyen d'une matière modelable (3) qui est disposée entre ladite surface et un côté d'une membrane déformable (2), susceptible d'épouser la forme de ladite surface sous l'effet de la pression qu'y exerce ledit objet (4) ou ladite partie du corps, dispositif caractérisé en ce qu'il comporte des moyens pneumatiques commandés qui sont agencés de l'autre côté de ladite membrane (2) pour délivrer une contrepression continûment variable permettant de contrôler en permanence l'amplitude des déformations de ladite matière modelable (3).

5 - Dispositif pour l'obtention d'une empreinte selon la revendication 4, caractérisé en ce que les moyens pneumatiques commandés sont constitués par une poche flexible fermée à volume variable dans laquelle peut être introduit un fluide compressible.

6 - Dispositif pour l'obtention d'une empreinte selon la revendication 5, caractérisé en ce que le fluide compressible pouvant être introduit dans la poche flexible est un gaz ou un mélange gazeux.

7 - Dispositif pour l'obtention de l'empreinte de la voûte plantaire du pied d'une personne, au moyen d'une matière modelable (3) qui est disposée entre ladite voûte et un côté d'une membrane déformable (2) susceptible d'épouser la forme de ladite voûte sous l'effet de la pression qu'y exerce ledit pied, dispositif caractérisé en ce qu'il comporte au moins une chambre à air (1 ) à volume variable qui est agencée de l'autre côté de ladite membrane (2), et qui est reliée à un moyen de gonflage, ou gonfleur (5), muni d'un manomètre (7) de sorte à faire varier, tout en la contrôlant, la pression régnant dans ladite chambre (1).

8 - Dispositif pour l'obtention de l'empreinte de la voûte plantaire du pied d'une personne selon la revendication 7, caractérisé en ce que chaque chambre à air (1) est susceptible d'être gonflée entre une pression minimale et une pression maximale dont le rapport est supérieur ou égal à 20.

9 - Dispositif pour l'obtention de l'empreinte de la voûte plantaire du pied d'une personne selon la revendication 8, caractérisé en ce que chaque chambre à air (1) est susceptible d'être gonflée entre une pression minimale égale à 2 000 Pa et une pression maximale égale à 40 000 Pa.

10 - Dispositif pour l'obtention de l'empreinte de la voûte plantaire du pied d'une personne selon l'une quelconque des revendications 7 à 9, caractérisé en ce qu'il comporte deux chambres à air (1), interne et externe, maintenues côte à côte, chaque chambre (1) pouvant être gonflée de manière autonome pour solliciter la membrane déformable (2) symétriquement par rapport à son axe médian, ce qui procure un moyen pour contrebalancer séparément la pression exercée par la partie interne et la partie externe dudit pied sur ladite membrane (2).

11 - Dispositif pour l'obtention de l'empreinte de la voûte plantaire du pied d'une personne selon la revendication 10, caractérisé en ce que les deux chambres à air (1), interne et externe, sont constituées par des tores ou des secteurs toriques de révolution, de sections droites circulaires, ovales ou oblongues, normalement prises identiques, le rayon intérieur de la chambre (1) placée du côté proximal du pied étant au moins égal à 40 centimètres, le rayon extérieur de la chambre (1) placée du côté distal du pied étant au moins égal à 65 centimètres, et la longueur de l'arc séparant les deux chambres (1) étant au moins égale à 40 centimètres.

12 - Dispositif pour l'obtention de l'empreinte de la voûte plantaire du pied d'une personne selon l'une quelconque des revendications 7 à 9, caractérisé en ce qu'il comporte une seule chambre à air (1) constituée par un tore de révolution, de section droite circulaire, ovale ou oblongue, dont le rayon intérieur est au moins égal à 40 centimètres et dont le rayon extérieur est au moins égal à 65 centimètres.

13 - Dispositif pour l'obtention de l'empreinte de la voûte plantaire du pied d'une personne selon l'une quelconque des revendications 7 à 9, caractérisé en ce qu'il comporte une seule chambre à air (1) constituée par un secteur torique de révolution, de section droite circulaire, ovale ou oblongue, dont le rayon intérieur est au moins égal à 40 centimètres, dont le rayon extérieur est au moins égal à 65 centimètres, et dont la longueur moyenne, prise au niveau de l'arc médian des arcs intérieur et extérieur, est au moins égale à 40 centimètres.

14 - Dispositif pour l'obtention de l'empreinte de la voûte plantaire du pied d'une personne selon la revendication 13, caractérisé en ce que la membrane déformable (2), sur laquelle est posée la matière modelable (3) destinée à reproduire la surface de ladite voûte plantaire, est tendue devant un ajourage oblong (9) qui est pratiqué dans la face supérieure d'un caisson (8) contenant la chambre à air (1) en forme de secteur torique, l'axe médian dudit ajourage (9) étant orienté sur ledit caisson (8) pour être sensiblement tangent au milieu de l'arc médian des arcs intérieur et extérieur de ladite chambre (1).

15 - Dispositif pour l'obtention de l'empreinte de la voûte plantaire du pied d'une personne selon la revendication 14, caractérisé, d'une part, en ce que la matière modelable (3) destinée à reproduire la surface de ladite voûte plantaire est une matière thermoformable pour semelles, et, d'autre part, en ce que l'ajourage oblong (9) est plus grand que la semelle correspondant à la pointure de pieds la plus élevée.

16 - Dispositif pour l'obtention de l'empreinte de la voûte plantaire du pied d'une personne au moyen d'une semelle thermoformable de base selon la revendication 15, caractérisé en ce que des éléments de correction (10), thermoformés ou non, sont intercalés entre ladite semelle de base et la membrane déformable (2).

17 - Dispositif pour l'obtention de l'empreinte de la voûte plantaire du pied d'une personne au moyen d'une semelle thermoformable de base selon l'une quelconque des revendications 15 ou 16, caractérisé en ce que la zone de la membrane déformable (2) délimitée par l'ajourage oblong (9) est pourvue d'un repérage longitudinal millimétrique pour le positionnement de ladite semelle de base et/ou des éléments de correction (10).

18 - Appareillage pour la fabrication de semelles anatomiques ou correctives adaptées aux voûtes plantaires des deux pieds d'une personne, caractérisé en ce qu'il comporte, pour la fabrication de chacune des semelles, un dispositif selon l'une quelconque des revendications 15 à 17, ces deux dispositifs étant montés dans un support commun (11) et pouvant être écartés l'un de l'autre pour tenir compte de l'écartement des deux pieds.

19 - Appareillage selon la revendication 18, caractérisé en ce qu'il comporte deux cannes verticales amovibles permettant à la personne de se tenir lorsqu'elle se trouve en appui, par ses deux pieds, sur les membranes déformables (2) des caissons (8) équipant respectivement les dispositifs pour l'obtention de l'empreinte de la voûte plantaire de chaque pied.

20 - Appareillage selon l'une quelconque des revendications 18 ou 19, caractérisé en ce que les axes médians des ajourages oblongs (9) des deux caissons (8) équipant respectivement les dispositifs pour l'obtention de l'empreinte de la voûte plantaire de chaque pied sont orientés de 30 ⁰ l'un par rapport à l'autre.
